# EUROPEAN PATENT SPECIFICATION

(11) **EP 4 275 740 B1**
(45) Date of publication and mention of the grant of the patent: **04.11.2026**
(21) Application number: 22172464.4
(22) Date of filing: 10.05.2022
(51) Int. Cl.: A61N 5/10

(54) **RADIOTHERAPY TREATMENT PLANNING AND MEANS FOR RADIOTHERAPY TREATMENT DELIVERY**
PLANUNG VON STRAHLENTHERAPIEPLANUNG UND MITTEL ZUR DURCHFÜHRUNG VON STRAHLENTHERAPIEBEHANDLUNGEN
PROCÉDÉ DE PLANIFICATION DE TRAITEMENT DE RADIOTHÉRAPIE ET MOYEN D'ADMINISTRATION D'UN TRAITEMENT DE RADIOTHÉRAPIE

(43) Date of publication of application: 15.11.2023
(73) Proprietor: RaySearch Laboratories AB, 104 30 Stockholm (SE)
(72) Inventor: TRANEUS, Erik, 752 39 UPPSALA (SE)

(56) References cited:
- WO-A1-2021/094824
- US-A1- 2009 180 589
- US-A1- 2011 166 407
- US-A1- 2020 346 034

## Description

### Technical Field

The present invention relates to radiotherapy treatment planning involving any type of radiotherapy treatment, including photons or charged particles such as electrons or photons or other ions, and to apparatus and computer program for such treatment planning. It also relates to apparatus and computer program for delivery of radiotherapy treatment.

### Background

A main aim of radiotherapy treatment planning disclosed in US 2009/0180589 A1 and US 2020/0346034 A1, for instance, is to devise a plan that will ensure a desired dose to a target such as a tumor and a low dose to healthy tissue. At the same time, any particularly sensitive organ or tissue near the target may require zero dose or as close to zero as possible. Typically, radiotherapy involves giving a total dose divided into a number of portions, often called fractions. When delivering dose by conventional techniques delivery of one fraction takes of the order of a minute or so.

In some cases, the part of the patient that is being treated will move during a treatment fraction. For example, treatment of the thorax will be affected by the patient's breathing. Various techniques exist for handling this including robust planning, following the movement of the target, or restraining breathing for certain periods of time. A breathing cycle normally takes in the order of magnitude of 10 s. Another method involves gating the beam in view of the movement so that the beam will be turned on only in the periods when the target and any risk organs are in well-defined positions, for example at the end of each exhalation, or while the patient is holding their breath.

In radiotherapy treatment the heart can be either part of the treated volume or a risk organ, for example for treating tumors in the lung or chest, or breast cancer. In both these cases the heart's movement poses specific problems. In the former case, to calculate the accumulated dose to a specific part of the heart means tracking that part throughout each heartbeat. In the latter case, the heart as an organ at risk should be avoided as much as possible, ideally to receive no dose at all.

A typical frequency for the heart is approximately 1 Hz, which means that the dose to a particular position in the heart is the accumulated result of the delivery timing details and the heart's beating motion. An arbitrary point in the heart can move in the order of magnitude of a few centimeters over a heartbeat. Traditionally, the effects of this movement are handled in one of the following ways.

One way of accounting for the movement of the heart is as follows: The heart is imaged by a cardiac-CT where a series of images are acquired over a heartbeat cycle. Typically, there is one image per 100 ms over a 2 second cycle. This is analogous with standard 4dCT images series used for treatment planning in e.g. the thorax region (lung) where each image covers a 1 second interval with ten such phases in total. This intricate accumulation can be analyzed through so called interplay evaluation. Alternatively, or in addition, the effect of the heart motion delivery dynamics can be reduced by robust optimization techniques. Both are complex and time consuming.

### Summary of the invention

It is an object of the present disclosure to provide a radiotherapy treatment planning method and system that will result in plans that compensate for the movements involved in a heartbeat in a less complex way than the prior art.

The invention is defined in the appended set of claims.

One aspect of the present disclosure relates to a computer-implemented method of obtaining a radiation therapy treatment plan for a patient, including the following steps:
- obtaining a 4D image of a heartbeat
- deriving from the 4D image a treatment phase of the heartbeat in which radiation should be delivered to the patient,
- obtaining an optimization problem, said optimization problem defining planning objectives, including information about the treatment phase,
- optimizing the values of a set of planning parameters to achieve the planning objectives, in such a way that the radiation will be delivered only during the treatment phase.

The planning parameters typically also include standard planning parameters, which will depend on the treatment modality. For example, for proton-based Pencil Beam Scanning (PBS), the planning parameters also include standard planning parameters such as spot weights. The planning objectives are typically defined to ensure uniform dose to a target while sparing other tissues and in particular organs at risk. The step of obtaining an optimization problem may include formulating an optimization problem based on the current patient anatomy, or using a pre-formulated optimization problem.

The set of planning parameters may also include model parameters defining the properties of a modulating device that is to be used to modulate the beam during delivery. Such a modulating device enables conformal treatment while using only a single energy layer, since it is arranged to modulate the beam energy in different ways in different sub-areas across the beam area.

In some preferred embodiments, the optimization step is performed in such a way that the radiation will be delivered during the treatment phase of only one heartbeat. Because or the short delivery times involved, this normally requires dose rates that are higher than the dose rates that are currently normally used, but such higher dose rates are also achievable with standard equipment. Because of the short delivery times, interplay effects and other issues with organ motions will be eliminated or significantly reduced.

In other preferred embodiments, the optimization step is performed in such a way that the radiation will be delivered during the treatment phase of two or more heartbeats. This will enable the changes between treatment phases, which cannot typically be made within one heartbeat, such as changes in energy layer and/or gantry angle. Accordingly, the treatment may be delivered from one direction, or from two or more directions.

Obtaining a suitable optimization problem and using it to optimize the compensating device enables a fast and reliable design of a compensating device as defined. It also enables other advantageous aspects to be considered in the design process, for example robustness, linear energy transfer (LET) based objectives or relative biological effect (RBE) based objectives.

The present disclosure also relates to a computer program product comprising computer-readable code means which, when run in a computer, will cause the computer to perform the planning method as discussed above, and to a computer system for running such a computer program. The computer program product may include a non-transitory storage medium whereon the code means are stored.

The present disclosure also relates to a radiotherapy delivery system and to a computer program product comprising computer-readable code means for controlling such a delivery system. The computer program product may include non-transitory storage means on which the code means is stored.

Such a computer program product controlling delivery of a fraction of a radiotherapy treatment plan to a patient, is arranged to perform the following steps:
- receive heartbeat data related to the heartbeat rhythm of the patient
- identify based on said heartbeat data a time period within a heartbeat as a treatment phase in which radiation should be delivered
- control the delivery so that radiation is only delivered to the patient during the identified treatment phase of one or more heartbeats.

The computer program product may be arranged to control the delivery of the fraction during the identified treatment phase in one heartbeat, or over the treatment phases in two or more heartbeats. If the radiation is delivered in the treatment phase of only one heartbeat, the dose rate must be adapted so that the whole fraction dose can be delivered within a very short time, in the order of magnitude of 1/10 s.

The method according to the present disclosure may be applied to any radiotherapy treatment modality, including photons or charged particles. The charged particles may be electrons or ions. The most common form of ion-based radiotherapy is proton therapy.

The disclosure also relates to a radiotherapy delivery system for delivering a radiotherapy treatment plan, said radiotherapy delivery system comprising a data memory holding a treatment plan obtained according to the method defined above, and a control unit arranged to control the radiotherapy delivery system according to the treatment plan.

### Brief description of drawings

The invention will be described in more detail in the following, by way of examples and with reference to the appended drawings.
Fig. 1 is a flow chart of a treatment delivery method according to the present disclosure.
Fig. 2 is a flow chart of a treatment planning method according to the present disclosure.
Fig. 3 is a schematic drawing of a beam modulating device that may be used in the context of the methods described herein.
Fig. 4 is a schematic drawing of a computer system that may be used for treatment planning.
Fig. 5 is a schematic drawing of a treatment delivery system that may be used in the context of the present disclosure.

### Detailed description of embodiments

The method according to the present disclosure is based on the use of ECG data, or other suitable data related to the heart rhythm, for gating beam delivery to a patient. This means that each beam must be delivered in a short amount of time, of significantly less than one second. Different ways to implement this will be discussed in the following. Some embodiments of the method rely on the beam being delivered at a dose rate that is significantly higher than conventional dose rates, so that a whole fraction dose can be delivered in a fraction of a second.

As will be discussed in the following, the method is based on determining a treatment phase of each heartbeat, which is the window in time in which radiation should be delivered to the patient. This treatment phase may be selected so that the radiation will avoid the heart, or to treat a specific portion of the heart. In both cases, the movement of the heart must be taken into account.

Delivery may in some embodiments be completed within the timeframe of just one heartbeat. In that case the delivery must be made at a sufficiently high dose rate to deliver the full fraction dose within just one treatment phase. A full heartbeat is normally at the order of magnitude of 1 s and the phase in which it is suitable to deliver the dose will be a fraction of that, for example 1/10, that is the order of magnitude of 0.1 s. If the desired fraction dose is 2 Gy, this means that the dose rate must be 20 Gy/s.

Alternatively, the fraction dose may be delivered in several portions, gated so as to be delivered in the treatment phases of two or more heartbeats, the beam being shut off during the remaining phases of the heartbeats.

Some embodiments employ a means of ensuring a conformal single energy layer dose distribution, if the dose is to be delivered in one go. This may not be necessary if the delivery machine is able to change energy layers within the timeframe of the treatment phase, that is the order of magnitude of 1/10 of a second. It may also not be necessary if the dose is distributed over several heartbeats so that the energy layers can be changed in between. For ion therapy such means could be for example a modulating device such as the one disclosed in EP20192106.1 by the same applicant. Such a modulating device is arranged to cause a beam to conform to the target without having to change the energy level of the beam.

Since the heart moves with the patient's breathing, information related to the movement of the thorax will also be needed in most cases. For the cases in which treatment is delivered in a very short time, where the position of the heart relative to the beam will be substantially unchanged, information about the position of the heart at the time of treatment, but not its movement, will be needed.

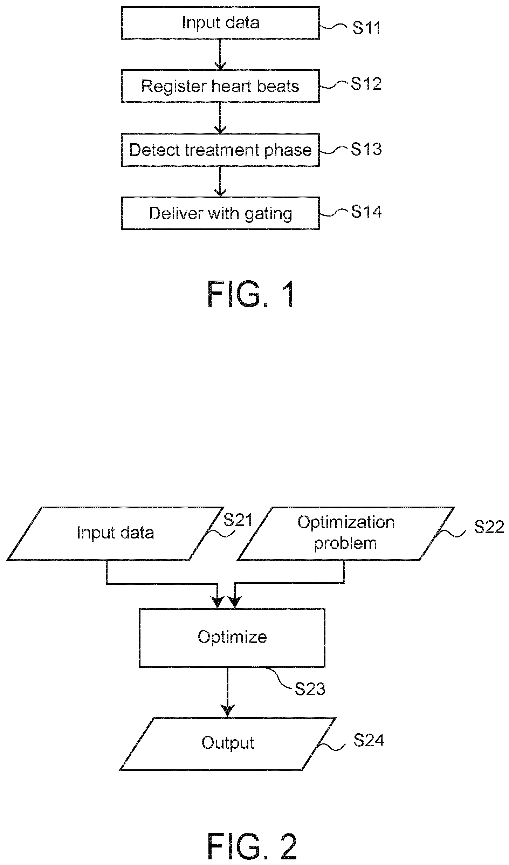

Fig. 1 illustrates a possible method for delivery of a treatment plan according to the present invention. Input data S11 includes a treatment plan arranged for delivery within the treatment phase of one or more heartbeats. In step S12, the patient's heart beats are registered, for example using an ECG. This is performed continuously. In the ECG signal, a point in time when the heart is in the right phase for delivering the beam is detected in step S13.

In step S14, gating is performed on the beam based on the detected point in time to ensure delivery of the beam to the patient in the right phase of the heartbeat.

Delivery in several portions over several heartbeats may be performed from the same direction but will typically involve more than one beam angle. Radiation is normally delivered from more than one beam angle, to reduce harm to healthy tissue. For proton therapy, or other radiotherapy treatment involving charged particles that will stop and deposit their energy in a desired position within the patient, the whole fraction dose can be delivered from fewer beam angles.

Therefore, the planning is based on one or more cardiac CT planning images. The phases in the heartbeat in which it is suitable to deliver the beam are identified and will be referred to as treatment phases. This is normally when the heart muscle contracts so that the geometric extension of the heart is small enough that the heart does not block the tissue that is to receive the beam. Beam gating is a well-known function in other areas, for example, based on the breathing pattern of the patient. The gating may also be performed to ensure that radiation reaches a specific portion of the heart, instead of avoiding it.

When the radiation modality is proton or other ion radiation, it is possible to deliver the whole dose within the treatment phase of one heartbeat. This requires that the dose rate is higher than conventional dose rates, which are normally less than or equal to approximately 1 Gy/s. A typical fraction dose is 2 Gy with conventional delivery times of approximately 30 - 60 s, which means that the average dose rate over the treatment volume is in the order of magnitude of 0.03 Gy/s - 0.07 Gy/s. To deliver such a dose within 1/10 s, the average dose rate has to be 20 Gy/s. This is unconventional but is achievable with available delivery systems.

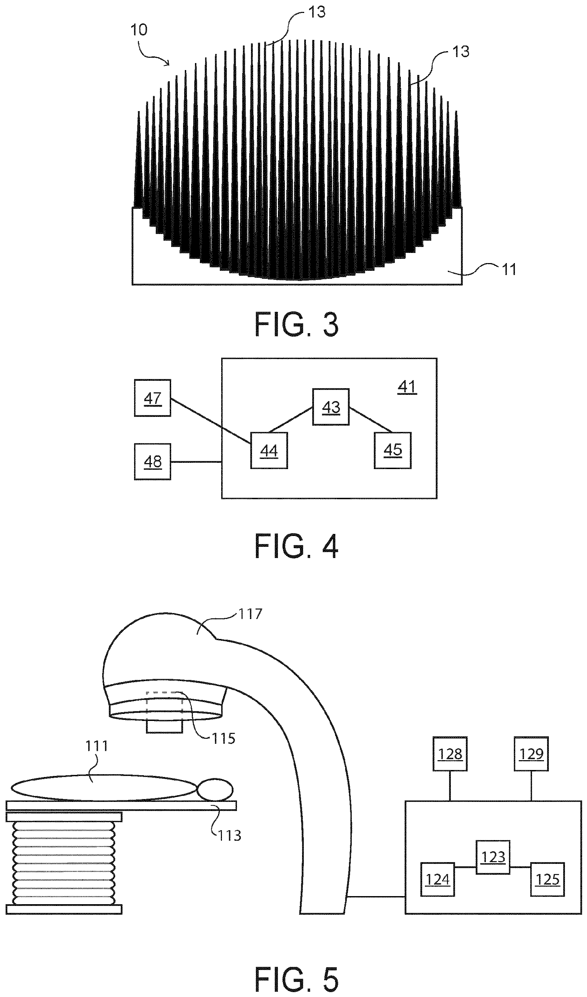

With most prior art proton delivery apparatuses, changing the energy layer takes approximately 0.5 s - 1 s, which means that it is impossible to change the energy layer during a delivery within a heartbeat. In such cases, an energy modulating device may be used to modulate the energy of the beam over the whole field so that the target will be covered with the desired dose. One example of such an energy modulating device will be described in the following with reference to Fig. 3. Another option would be to divide the fraction over several heartbeats, gating the delivery such that the beam is only on during the suitable part ("treatment phase") of each heartbeat, and changing the energy layer while the beam is off. In that way, several energy layers can be delivered over a short period of time and taking into account the movement of the heart that occurs with every heartbeat. The gated delivery may be delivered from the same beam angle in each heartbeat, or the beam angle can be changed so that at least some beams will be delivered from different beam angles.

In the case of photon radiation, it is normally necessary to deliver each fraction as a number of beams from different angles, preferably an odd number such as 3, 5 or 7 beams, which intersect in the target. In that way, the dose to the target can be sufficiently high while surrounding tissue is spared. In this case the delivery may be gated so that each beam is delivered during the suitable part of a heartbeat.

The present disclosure is also applicable to electron radiation in the same way as for ions as discussed above. Electrons are typically used for treatment of targets located in the skin or at a maximum of 4 - 5 cm into the patient's body.

Figure 2 is a flow chart of a method that may be used for optimizing a treatment plan. Input data S21 to this method includes a set of planning parameters for the plan, and an optimization problem S22. The input data include standard planning parameters such as spot weights. The planning objectives of the optimization problem are typically defined to ensure uniform dose to a target while sparing other tissues and in particular organs at risk.

Initially, an optimization problem S22 to be used for optimizing the modulating device is also obtained. The optimization problem comprises objective functions and/or constraints related to the how the incoming radiation should be affected by the modulating device. The step of obtaining an optimization problem may include formulating an optimization problem based on the current patient anatomy, or using a pre-formulated optimization problem. The optimization problem may also be set up to include the design of the modulating device, as described in the aforementioned EP20192106.1.

In step S23, the optimization is performed, based on the optimization problem to yield a treatment plan S24.

The final optimization is performed with a single energy layer per beam. The final optimization can include one beam or multiple beams and any type of advanced objective function such as functions related to RBE dose, LET or robustness.

The energy modulating device is included to ensure that the entire dose per beam may be delivered by one single energy layer. As mentioned above, the whole fraction can then be delivered using only one beam during one treatment phase of the heartbeat, but it may also be distributed over several heartbeats, gated so as to only provide radiation during the treatment phase of each heartbeat. Alternatively, if the delivery system that is to be used when delivering the treatment to the patient is able to switch energy layers very fast, the optimization can be done with two or more energy layers for each beam.

Figure 3 discloses a passive modulation device 10 which may be used as an energy modulating device in the context of the present disclosure. The device 10 comprises a compensator element 11 which is essentially a disk having varying thickness across its area. The thickness is designed to cause an incoming radiation field to conform to the distal end of the target. On the disk a number of protrusions in the form of spike-shaped structures 13 are arranged, typically of the same material as the compensator element. The protrusions 13 are typically placed in a grid pattern on the disk, each occupying an area of, for example, 1.5x1.5 mm² of the disk. The protrusions 13 have different heights and shapes, selected in such a way that a beam passing through the device will be modulated to have Bragg peaks covering the whole of a target in a desired way. Of course, the size, shape and thickness of the compensator element, the arrangement of the protrusions on the compensator element and their size and heights, should be selected to conform to the target.

Further information about how to design such a passive modulation device and how to use it is found in EP20192106.1.

Figure 4 is a schematic overview of a computer system in which the optimization according to the invention may be carried out. A computer 41 comprises a processor 43, a data memory 44 and a program memory 45. Preferably, one or more user input means 47, 48 is also present, in the form of a keyboard, a mouse, a joystick, voice recognition means and/or any other available user input means. The user input means may also be arranged to receive data from an external memory unit.

A treatment plan is found in the data memory 44. The treatment plan may be generated in the computer 41, or received from another storage means in any way known in the art. The data memory also comprises the characteristics of the dose delivery beam to be used in the actual treatment of the patient, that is, the beam that is to be modulated by the compensation device.

The data memory 44 also holds properties of the energy modulating device 10 such as its material composition, if a modulating device is to be used. If the material is known, its properties may be stored. As will be understood, the data memory 44 is only shown schematically. There may be several data memory units, each holding one or more different types of data, for example, one data memory for the design of the compensation device, etc..

The program memory 45 holds a computer program arranged to control the processor to perform the optimization procedure according to the invention. Like the data memory 44, the program memory may also be implemented as one or several units as is seen fit.

Figure 5 is a schematic drawing of a delivery system that may be used according to the invention, typically but not necessarily adapted to provide radiation to a patient from different directions. As will be understood, such systems may be designed in any suitable way and the design shown in Figure 5 is only an example. A patient 111 is positioned on a treatment couch 113. The system comprises a treatment unit 110 having a radiation source 115 mounted in a gantry 117 for emitting radiation towards the patient positioned on the couch 113. Typically, the couch 113 and the gantry 117 are movable in several dimensions relative to each other, to provide radiation to the patient as flexibly and correctly as possible. The gantry may be arranged to rotate around the couch, either between certain angles or a full 360 ° rotation. Alternatively, the gantry can be fixed and the couch carrying the patient can rotate instead. Another alternative is to have the patient in seated position in a chair. The arc treatment in this case could be performed by changing the beam directions or rotating the chair. These parts are well known to the skilled person. The system also comprises a computer 121 which may be used for radiotherapy treatment planning and/or for controlling radiotherapy treatment. As will be understood, the computer 121 may be a separate unit not connected to the imaging unit.

The computer 121 comprises a processor 123, a data memory 124, and a program memory 126. Preferably, one or more user input means 128, 129 are also present, in the form of a keyboard, a mouse, a joystick, voice recognition means or any other available user input means. The user input means may also be arranged to receive data from an external memory unit.

The data memory 124 comprises data such as clinical data and/or other information used to obtain a treatment plan, including a set of clinical goals to be used for planning. The data memory 124 also comprises one or more dose maps for one or more patients to be used in treatment planning according to embodiments of the invention. The program memory 126 holds a computer program, known per se, arranged for treatment plan optimization. The program memory 126 also holds a computer program arranged to make the computer control the treatment of a patient in accordance with the invention.

As will be understood, the data memory 124 and the program memory 126 are shown and discussed only schematically. There may be several data memory units, each holding one or more different types of data, or one data memory holding all data in a suitably structured way, and the same holds for the program memories. One or more memories may also be stored on other computers. For example, the computer may only be arranged to perform one of the methods, there being another computer for performing the optimization.

## Claims

1. A computer-implemented method of obtaining a radiation therapy treatment plan for a patient, including the following steps:
- obtaining a 4D image of a heartbeat,
- deriving from the 4D image a treatment phase of the heartbeat in which radiation should be delivered to the patient,
- obtaining an optimization problem, said optimization problem defining planning objectives, including information about the treatment phase,
- optimizing the values of a set of planning parameters to achieve the planning objectives, in such a way that the radiation will be delivered only during the treatment phase.

2. A computer-implemented method according to claim 1 wherein the set of planning parameters includes model parameters defining the properties of an energy modulating device that is to be used to modulate the beam energy during delivery.

3. A computer implemented method according to claim 1 or 2, wherein the optimization step is performed in such a way that the radiation will be delivered during the treatment phase of only one heartbeat.

4. A computer implemented method according to claim 1 or 2, wherein the optimization step is performed in such a way that the radiation will be delivered during the treatment phases of two or more heartbeats.

5. A computer implemented method according to claim 5, wherein the optimization step is performed in such a way that the radiation will be delivered from two or more directions.

6. A computer program product comprising computer-readable code means which, when run in a computer, will cause the computer to perform the method according to any one of the preceding claims.

7. A computer system for optimizing a radiotherapy treatment plan, said computer system comprising a program memory holding a computer program product according to claim 6 and a processor arranged to execute said program.

8. A radiotherapy delivery system for delivering a radiotherapy treatment plan, said radiotherapy delivery system comprising a data memory holding a treatment plan obtained according to the method of any one of the claims 1 - 5, and a control unit arranged to control the radiotherapy delivery system according to the treatment plan.

## Patentansprüche

1. Computerimplementiertes Verfahren zum Erhalten eines Strahlentherapiebehandlungsplans für einen Patienten, einschließlich der folgenden Schritte:
- Erhalten eines 4D-Bildes eines Herzschlags,
- Ableiten, von dem 4D-Bild, einer Behandlungsphase des Herzschlags, in der Strahlung an den Patienten abgegeben werden soll,
- Erhalten eines Optimierungsproblems, wobei das Optimierungsproblem Planungsziele definiert, die Informationen über die Behandlungsphase einschließen,
- Optimieren der Werte eines Satzes von Planungsparametern, um die Planungsziele zu erreichen, auf solche Weise, dass die Strahlung nur während der Behandlungsphase abgegeben wird.

2. Computerimplementiertes Verfahren nach Anspruch 1, wobei der Satz von Planungsparametern Modellparameter einschließt, die die Eigenschaften einer Energiemodulationsvorrichtung definieren, die zu verwenden ist, um die Strahlenergie während einer Abgabe zu modulieren.

3. Computerimplementiertes Verfahren nach Anspruch 1 oder 2, wobei der Optimierungsschritt auf solche Weise durchgeführt wird, dass die Strahlung während der Behandlungsphase von nur einem Herzschlag abgegeben wird.

4. Computerimplementiertes Verfahren nach Anspruch 1 oder 2, wobei der Optimierungsschritt auf solche Weise durchgeführt wird, dass die Strahlung während der Behandlungsphasen von zwei oder mehr Herzschlägen abgegeben wird.

5. Computerimplementiertes Verfahren nach Anspruch 5, wobei der Optimierungsschritt auf solche Weise durchgeführt wird, dass die Strahlung aus zwei oder mehr Richtungen abgegeben wird.

6. Computerprogrammprodukt, umfassend computerlesbare Codemittel, die, wenn sie auf einem Computer ablaufen, den Computer veranlassen, das Verfahren nach einem der vorstehenden Ansprüche durchzuführen.

7. Computersystem zum Optimieren eines Strahlentherapiebehandlungsplans, das Computersystem umfassend einen Programmspeicher, der ein Computerprogrammprodukt nach Anspruch 6 enthält, und einen Prozessor, der angeordnet ist, um das Programm auszuführen.
-

8. Strahlentherapieabgabesystem zum Abgeben eines Strahlentherapiebehandlungsplans, das Strahlentherapieabgabesystem umfassend einen Datenspeicher, der einen Behandlungsplan enthält, der gemäß dem Verfahren nach einem der Ansprüche 1 bis 5 erhalten wird, und eine Steuereinheit, die angepasst ist, um das Strahlentherapieabgabesystem gemäß dem Behandlungsplan zu steuern.

## Revendications

1. Procédé mis en œuvre par ordinateur pour l'obtention d'un plan de traitement de thérapie par rayonnements pour un patient, comportant les étapes suivantes :
- obtention d'une image 4D d'un battement de cœur,
- dérivation, à partir de l'image 4D, d'une phase de traitement du battement de cœur dans laquelle un rayonnement doit être administré au patient,
- obtention d'un problème d'optimisation, ledit problème d'optimisation définissant des objectifs de planification, comportant des informations concernant la phase de traitement,
- optimisation des valeurs d'un ensemble de paramètres de planification pour atteindre les objectifs de planification, de telle sorte que le rayonnement sera administré uniquement pendant la phase de traitement.

2. Procédé mis en œuvre par ordinateur selon la revendication 1, dans lequel l'ensemble de paramètres de planification comporte des paramètres de modèle définissant les propriétés d'un dispositif de modulation d'énergie qui doit être utilisé pour moduler l'énergie du faisceau pendant l'administration.

3. Procédé mis en œuvre par ordinateur selon la revendication 1 ou 2, dans lequel l'étape d'optimisation est réalisée de telle sorte que le rayonnement sera administré pendant la phase de traitement d'un seul battement de cœur.

4. Procédé mis en œuvre par ordinateur selon la revendication 1 ou 2, dans lequel l'étape d'optimisation est réalisée de telle sorte que le rayonnement sera administré pendant les phases de traitement de deux battements de cœur ou plus.

5. Procédé mis en œuvre par ordinateur selon la revendication 5, dans lequel l'étape d'optimisation est réalisée de telle sorte que le rayonnement sera administré à partir de deux directions ou plus.

6. Produit-programme d'ordinateur comprenant un moyen de code lisible par ordinateur qui, lorsqu'il est exécuté dans un ordinateur, amènera l'ordinateur à réaliser le procédé selon l'une quelconque des revendications précédentes.

7. Système informatique permettant d'optimiser un plan de traitement de radiothérapie, ledit système informatique comprenant une mémoire de programme contenant un produit-programme d'ordinateur selon la revendication 6 et un processeur configuré pour exécuter ledit programme.
-

8. Système d'administration de radiothérapie permettant d'administrer un plan de traitement de radiothérapie, ledit système d'administration de radiothérapie comprenant une mémoire de données contenant un plan de traitement obtenu selon le procédé de l'une quelconque des revendications 1 à 5, et une unité de commande configurée pour commander le système d'administration de radiothérapie selon le plan de traitement.
